# EUROPEAN PATENT APPLICATION

(11) **EP 3 992 847 A1**
(43) Date of publication of application: **04.05.2022**
(21) Application number: 20204105.9
(22) Date of filing: 27.10.2020
(51) Int. Cl.: G06K 9/46, A61M 1/06, G01F 23/02

(54) **A BOTTLE ANALYSIS SYSTEM**

(71) Applicant: Koninklijke Philips N.V., 5656 AG Eindhoven (NL)
(72) Inventor: KOCKX, Franciscus Nicolaas, 5656 AE Eindhoven (NL); JASCHKE, Lena Maria, 5656 AE Eindhoven (NL); SINGHVI, Priyanka, 5656 AE Eindhoven (NL)
(74) Representative: Philips Intellectual Property & Standards

(57) **Abstract**

A bottle analysis system receives image data of a bottle to be analyzed, and the data is processed to identify a shape of the bottle, and optionally any identifying markings. A bottle type is then determined. Image analysis is used to determine a liquid level in the bottle and thereby determine a liquid volume in the bottle.

## Description

### FIELD OF THE INVENTION

The invention relates to the analysis of bottles, and in particular bottles for feeding milk to an infant, or for collecting milk while using a breast pump.

### BACKGROUND OF THE INVENTION

There are many different types, shapes and sizes of feeding bottle.

It is known that is it is of interest for mothers to monitor the amount of milk their baby is drinking, when feeding from a bottle, and to monitor the amount of milk collected during use of a breast pump. The volume of milk consumption and type of bottle used are important for born babies and toddlers. Over consumption or under consumption of milk are common problems faced by caregivers and parents alike. Studies have shown that overfeeding leads to vomiting, diarrhea, loss of weight, and in some cases death. Underfeeding leads to excessive jaundice, severe dehydration and hypoglycemia which can potentially cause brain injury.

With constant use and washing, the volume markings on feeding bottles tend to fade away, making the manual assessment of volume difficult. People also often find it hard to discern between different bottle types.

The applicant has proposed a system for automatic milk management using a sleeve which fits around a bottle, and incorporates a weighting scale to weigh the bottle before and after feeding the baby, thereby to determine the actual feeding volume. This system is disclosed in WO 2019/030029.

This requires an additional component which the user must employ. This component also needs to be designed to fit a multitude of bottle sizes and shapes.

There is a need for a simpler system for enabling monitoring of consumption volumes, or volumes produced during use of a breast pump.

### SUMMARY OF THE INVENTION

The invention is defined by the claims.

According to examples in accordance with an aspect of the invention, there is provided a bottle analysis system, comprising:
an input for receiving image data of a bottle to be analyzed; and
a processor, wherein the processor is adapted to:
   use image analysis to identify at least shape characteristics of the bottle, and thereby determine a bottle type;
   obtain further shape characteristics of the bottle from a database which relates different bottle types to said further shape characteristics; and
   use image analysis to determine a liquid level in the bottle and thereby determine a liquid volume in the bottle based on the bottle type and the further shape characteristics.

The image data is for example an image, multiple images or a video stream.

This system uses image analysis to identify a bottle type. In this way, the system does not need to fit to a particular bottle, but it can identify the bottle at least from its shape. The shape is for example obtained based on aspect ratios and other shape features, and preferably does not rely on absolute dimensions.

The bottle type is for example determined by accessing a database of pre-stored bottle types. For these pre-stored bottles, the bottle volume may be stored as data, as well as the volume for different liquid levels from the base of the bottle, or indeed a function relating the liquid level to the corresponding volume. This information may be considered to comprise further shape characteristics. For example, the initially identified shape characteristics may be scale independent, whereas the further shape characteristics relate to the specific scale of the bottle. In this way, a liquid level observed by image analysis can be converted simply into a volume based on the known 3D shape of the bottle, once its type has been identified.

The processor may further be adapted to use the image analysis to identify if any of a predetermined set of identifying markings is present. There are predetermined markings that are known to be present on certain bottles, such as brand names, or bottle type identifiers. The image analysis thus looks for these identifiers as well as the general bottle shape. However, these identifiers do not need to be applied to the bottles for the purposes of the system. Instead, they are, for example, existing surface marking features which form part of the standard bottle designs to be recognized by the image analysis.

If a pre-recognized bottle type cannot be identified, bottle shape characteristics may instead be identified based on analysis of the 3D shape.

By determining a liquid volume at multiple time points, the volume of liquid, especially milk, can be tracked. The processor for example uses computer vision techniques aided by pattern recognition and neural networks (which may be considered to comprise the implementation of artificial intelligence) as well as mathematical and statistical formulas and techniques.

The invention thus provides ease of integration with existing applications and enables a hassle-free baby feed monitoring experience. It is simple to share historical data between multiple users (e.g. multiple caregivers) so that they may keep track of the milk consumed by the infant.

By determining at least a shape of the bottle using image analysis, there is no need for special identifying markers and there is no need for the bottle to be positioned with any particular orientation relative the image capture system. The shape information does not vary with aging, such as with surface scratches, or color changes (due to wear). The same bottle type may also be recognized even if different different caps are used. The image recognition may be able to recognize the bottle type and the liquid level in a single step.

If a video stream is received as input as the image data, it is for example converted into separate images for subsequent image analysis.

The processor is for example adapted to determine a bottle type using a neural network. The neural network may be trained with existing bottles, and this may include bottles of multiple manufacturers. The training is also based on the identifying markings.

The processor may be adapted to use image analysis to:
identify a liquid surface; and optionally
identify liquid level markers on a surface of the bottle.

The liquid surface may be used to determine a volume, either based on a known volume to liquid level function for the particular bottle (as explained above), or by identifying alignment with liquid level markers (or both).

The processor may be adapted to process first image data and a second image data and determine a change in liquid volume. This enables consumption to be monitored over time, or indeed allows an amount of milk expressed to be measured in the case of use of a breast pump.

In one preferred implementation, the system is for monitoring bottle feeding of an infant, so that the changes in liquid volume correspond to milk consumption volumes.

The system may further comprise a memory for storing historic milk consumption volumes. This enables a mother or other caregiver to keep track of the feeding performance of the infant over time.

The processor may be adapted to determine an amount of milk to be fed to an infant based on the historic milk consumption volumes, and output the determined amount. This provides guidance to the feeding mother or caregiver of the suitable amount to feed the infant.

The processor may be adapted to output the determined amount by generating an augmented reality image of the bottle which represents the determined amount of milk. This provides an easy to follow direction for the mother or caregiver. In particular they can fill the bottle to the level as represented by the augmented reality.

The system may comprise a camera for capturing the images or video stream. In one example, the system is implemented as a mobile phone or tablet and hence uses the existing camera functionality as well as the existing processing capability.

The invention also provides a method of an analyzing a bottle, comprising:
receiving image data of the bottle to be analyzed;
using image analysis to identify at least shape features of the bottle, and thereby determine a bottle type;
obtaining further shape characteristics of the bottle by accessing a database which relates different bottle types to said further shape characteristics; and
using image analysis to determine a liquid level in the bottle and thereby determine a liquid volume in the bottle based on the bottle type and said further shape characteristics.

This method may be applied to a range of bottle types and sizes. It is simple for the user, in that they simply have to capture images or video of the bottle, optionally at different points in time if changes in liquid volume are to be determined.

The method may comprise using the image analysis to identify if any of a predetermined set of identifying markings is present.

The method may comprise:
determining a bottle type using a neural network; and
using image analysis identify a liquid surface and optionally also to identify liquid level markers on a surface of the bottle.

The method may comprise processing first image data and second image data and determining a change in liquid volume. The method is for example for monitoring bottle feeding of an infant, wherein the changes in liquid volume correspond to milk consumption volumes, wherein the method further comprises determining an amount of milk to be fed to an infant based on historic milk consumption volumes, and outputting the determined amount.

The determined amount may be output by generating an augmented reality image of the bottle which represents the determined amount of milk.

The invention also provides a computer program to implement the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a baby bottle and also a captured image of the baby bottle on the screen of a smart phone;
Figure 2 shows a first method of analyzing a bottle; and
Figure 3 shows a development of the approach of Figure 2 in which the system establishes how much milk to provide in the bottle based on past feeding data of the baby.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a bottle analysis system in which image data (one or more images or a video stream) of a bottle to be analyzed are received, and they are processed to identify a shape of the bottle and any identifying markings. A bottle type is then determined and its characteristics may be obtained from a database. Image analysis is used to determine a liquid level in the bottle and thereby determine a liquid volume in the bottle.

To use the system, a bottle such as an infant feeding bottle, is photographed or filmed, for example before and after the feeding by the user. Empty bottles may also be photographed. The system may for example be implemented by software provided on a smartphone.

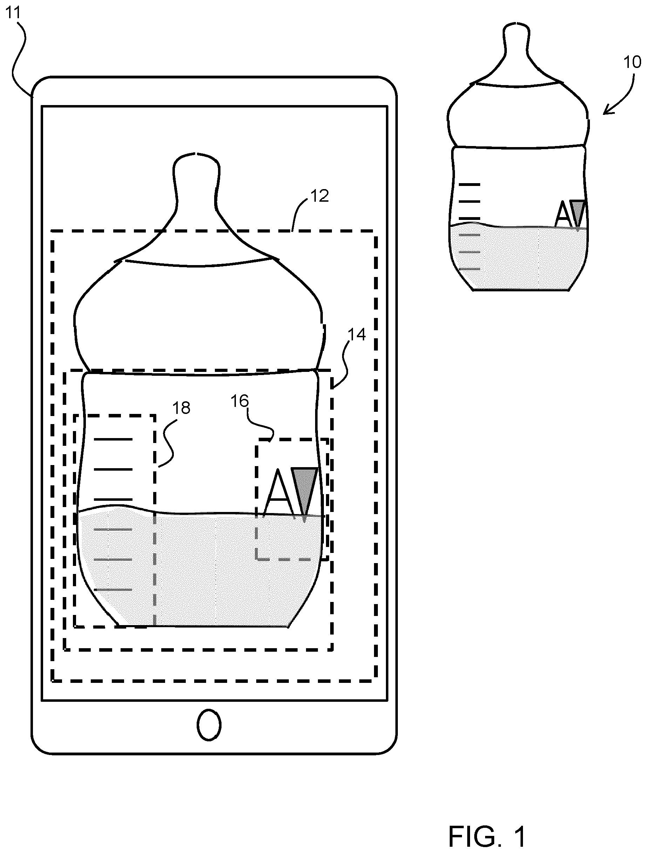

Figure 1 shows a baby bottle 10 and also a captured image of the baby bottle on the screen of a smart phone 11. Using any combination of neural networks, computer vision and mathematical techniques, the bottle type and the actual volume of liquid inside it may be determined. This is used to simplify the administration and tracking of baby feeds, while avoiding the need for a separate dedicated physical device or constant manual monitoring.

Figure 1 shows various regions of interest identified based on image processing, including the overall shape of the bottle within the area 12, the shape of the base part of the bottle in area 14, a marking such as a brand name and/or model type in area 16 and level markings in area 18.

By obtaining information from all these sources (when available - there may be no visible markings in many cases), the type of bottle may be determined from a database of previously analyzed bottles. This step may for example be carried out with the bottle empty or with any quantity of liquid. The database gives further information which was nolt obtained from the image processing, in particular so that the initial image processing does not need to be scaled. At this stage, the type of the bottle, the overall volume of the bottle, and the brand have been established from the database.

This information may then be provided to the user, for example so that they know they are using the correct bottle type before filling with milk.

The liquid level may also be established by recognizing the liquid boundary, or else it may be determined that the bottle is empty at this stage. The areas of interest may be identified in static images, or in live video, or in a set of images extracted from the video stream.

The determination is based on object detection techniques using computer vision and pattern recognition, including pre-processing of the images. The object detection is performed based on training of a neural network using internally created and labelled data and image processing.

A volume calculation involves finding the liquid boundary in the bottle followed by post processing techniques. Based on the known bottle type, a measured fraction of the bottle height corresponds to a known volume.

If a pre-stored bottle type is not available, the image processing may determine the 3D shape of the bottle (e.g. assuming a given wall thickness) and then mathematically derive the function of internal volume versus liquid height. The volume can in this way be determined by post processing techniques using image algorithms and/or mathematical formulas.

This may require additional images to those required to recognize a pre-stored bottle type. Thus, if the system is not able to recognize a known bottle type, it may instruct the user to take multiple images from a set of viewing angles to enable a 3D shape determination to be performed.

However, for volume calculation, scaled information, i.e. an absolute dimension, is then needed. This could for example be achieved by requiring the user to apply a scale to the bottle, to be captured by the image processing.

However, the preferred approach is for the algorithm to be trained and updated with any new bottle types. In this case, the system always relies upon recognizing a bottle type and then deriving volume information from a database.

The liquid volume may thus be determined by calculating the volume using mathematical techniques, based on a measurement of the ratio of the liquid height in the bottle to the actual bottle height, for example using bounding techniques and/or line detection algorithms. The ratio of liquid height to bottle height in insensitive to scale so once the bottle type is known, the absolute measurement of liquid level is not needed.

Alternatively, a template overlay can be used to determine the volume of the bottle by matching the detected volume against a fitted template on the frame or image of the baby bottle.

Due to the transparency of the bottles and constantly changing and dynamic backgrounds, a post-processing step may be used to add the required accuracy to the detection process. If the bottle is held in a tilted fashion, the bottle type can still easily be detected but the determination of volume in the presence of a liquid inside is not as accurate.

Thus, the user may be instructed to perform the analysis with the bottle standing on a horizontal surface such as a table.

Once the volume is known, the volume information can be presented and stored for the user and/or for further research purposes.

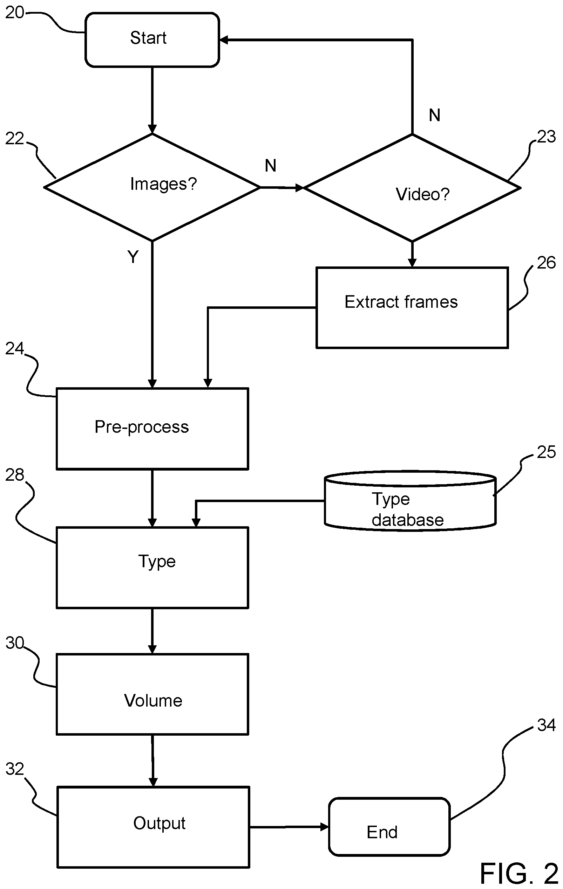

Figure 2 shows a method of analyzing a bottle. The method starts in step 20, during which images or a video stream is received of the bottle to be analyzed.

In step 22, it is determined if the received input comprises images, and if so the method proceeds to a pre-processing step 24.

If the input does not comprise individual images, it is determined in step 23 if a video stream has been received. If so, individual frames are extracted in step 26 before proceeding to the pre-processing step 24. If the input is also not video, no suitable input has been received, and the method returns to the start.

The pre-processing for example involves applying grayscale, removing excess noise and extracting the position of the bottle with respect to the image including the teat and the base.

After the pre-processing, the type of bottle is determined in step 28 using image analysis. This involves at least identifying a shape of the bottle, and also any identifying markings which are present. A bottle type for example has a particular shape of the base as well as a particular shape of the closing rim through which the teat projects. The bottle type is determined with reference to a bottle type database 25.

The bottle type is also supplemented with additional information from the database, and this additional information may be considered to be further shape characteristics, and they take into account the absolute dimensions of the particular identified bottle type.

In step 30, image analysis is used to determine a liquid level in the bottle and thereby determine a liquid volume in the bottle. This involves identifying the line caused by the liquid surface against the inner wall of the bottle. The line is preferably flat and straight, if the bottle is stationary on a flat surface. However, a tilt angle may be detected and based on the known 3D shape from the type database, a liquid volume may still be determined. The volume information is output to a user in step 32 before the method ends in step 34.

The system and method is for example implemented as a feature of bottle feed tracking app. The system and method may be used to aid feed monitoring and bottle detection in the absence of the user's ability to do so correctly. The tracking of volume over time may be used as part of the monitoring of the development of a baby. This may be used in hospital environments to automate and reduce the workload of tracking feed data.

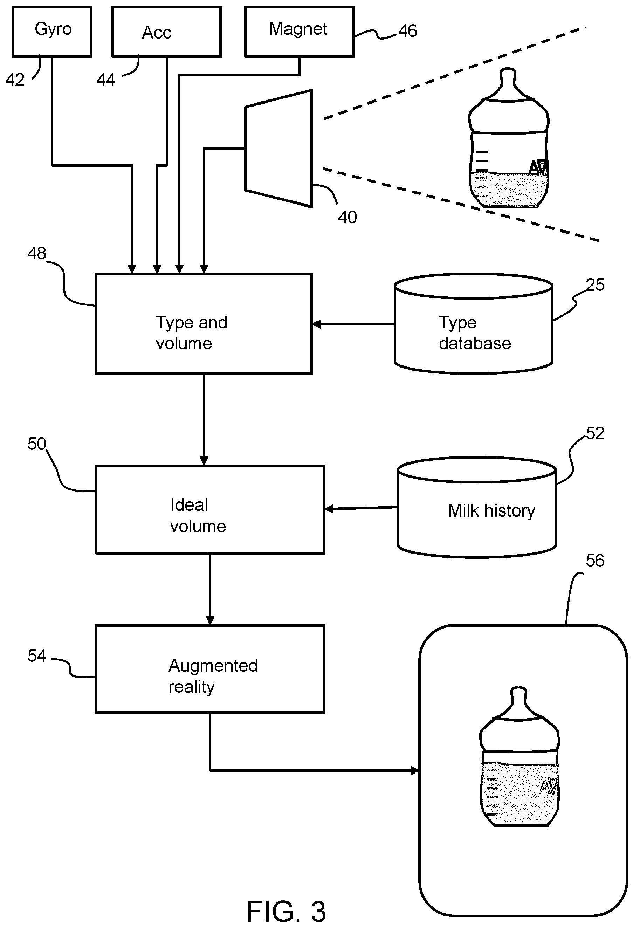

Figure 3 shows a development of the approach of Figure 2 in which the system establishes how much milk the user should provide in the bottle based on past feeding data of the baby.

In the particular example shown, the amount of milk is presented to the user using augmented reality. This enables a feed monitoring app to be made personalized for an individual baby. Users change bottles as babies grow, but the bottle recognition approach explained above is sufficiently adaptive to include all bottles and development stages. This will not only help in keeping a record of how much milk the baby has consumed and should consume but also can be used as an aid to record how much breast milk has been pumped for future use, and by when it should be fed to the baby.

Figure 3 shows the camera 40 as used in the example above, and also shows other sensors of a typical mobile phone which may also be employed in the processing method, including gyroscopes 42, accelerometers 44 and a magnetometer 46.

All of this sensor information is provided to the processor which performs the step 48 of determining the bottle type and volume, using the type database 25, in the same way as explained above.

The sensors are used to determine the position of the bottle relative to the mobile phone (or other camera device), for example based on the assumption that the bottle is positioned vertically. However, if the bottle is oriented non-vertically, this can also be detected based on identifying the bottle axis. By knowing the orientation of the camera, and the bottle axis as viewed in the camera image, a non-vertical bottle orientation can also be determined.

The magnetometer 46 is used to determine the true magnetic north of the mobile phone.

The accelerometer 44 is then used to know the orientation of the mobile phone e.g. portrait or landscape with respect to the gravity vector. This enables a base to be established for the real world, and the virtual world (for the augmented reality) with respect to the real world.

The gyroscope 42 is used to reduce jitter and the noise in the camera sensor and thereby make the positioning more accurate between the real world view and the virtual world.

The additional sensor data used in the processing step 48 may be used to compensate for angles of the camera. For example, a transparent bottle and level of the volume of milk therein may result in detection of multiple volume levels due to the transparency showing the front and back milk level. Knowing the angle at which the image is taken can help to determine which level to choose during level detection.

Additionally, the sensors can be used as an additional input for the algorithm, improving correct bottle classification. Thus, the use of sensors may improve the capability to accurately detect the bottle type and volume, as well as to implement the image overlay in the case of an augmented reality system, as described above.

In step 50, a milk history database 52 is accessed which stores historical feeding information about the individual baby. This is used to determine a desired milk volume to be fed to the baby.

The use of augmented reality for example involves the capture of a video sequence of the bottle. The determined desired milk quantity may then be prepared as an augmented reality overlay in step 54. The live images of the bottle are then applied to 3D rendering software, where the bottle is superimposed with the desired quantity of milk as determined based on the historical data.

The rendered graphic is then sent for post-processing where image algorithms for example provide a resolution adaptation to the resolution with which the image was captured, and provide suitable image processing to be augmented with the real world image or frames.

The graphic render is then provided with the real-world frame or image using a combiner.

Calibrated data from the sensors of the mobile device are in this way used in the rendering of the augmented reality. The use of sensors reduces the complexity of the image processing required, by providing additional context information to the images.

The final presented image 58 then shows in real time how much milk visually should be filled in the bottle.

The user may manually enter drinking information to the milk history database, if the automated volume monitoring was not used.

The camera may for example be stationary on a solid base such as a table with the display facing the user and with the camera facing the milk bottle. There could instead be a separate camera and display, for example the camera of a mobile phone and a separate display which are connected through standard communication means.

The visual feedback may include information other than the most suitable milk volume, such as an indication of the correct angle to hold the bottle while feeding the baby and so on.

The system may be used in a hospital environment, wherein instead of a nurse sitting with young parents, the system can be used to record and gives guidance on the bottle volume and other feeding characteristics. Furthermore, a recording can be analyzed afterwards by the doctor or other medical staff.

The system may be enhanced by adding labels to bottles. In this way, the system may be used to recognized individual bottles, and the system may then be enhanced to track bottles based on the dates they were filled, by providing a time stamp function. Thus, the system may have a milk management function.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

As discussed above, the system makes use of processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions. The skilled person would be readily capable of developing a processor for carrying out any herein described method.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

A single processor or other unit may fulfill the functions of several items recited in the claims.

A computer program may be stored/distributed on a suitable medium, such as an optical storage medium or a solid-state medium supplied together with or as part of other hardware, but may also be distributed in other forms, such as via the Internet or other wired or wireless telecommunication systems. (optional)

If the term "adapted to" is used in the claims or description, it is noted the term "adapted to" is intended to be equivalent to the term "configured to". The mere fact that certain measures are recited in mutually different dependent claims does not indicate that a combination of these measures cannot be used to advantage.

Any reference signs in the claims should not be construed as limiting the scope.

## Claims

1. A bottle analysis system, comprising:
an input for receiving image data of a bottle to be analyzed; and
a processor, wherein the processor is adapted to:
use image analysis to identify at least shape characteristics of the bottle, and thereby determine a bottle type;
obtain further shape characteristics of the bottle from a database which relates different bottle types to said further shape characteristics; and
use image analysis to determine a liquid level in the bottle and thereby determine a liquid volume in the bottle based on the bottle type and the further shape characteristics.

2. The system of claim 1, wherein the processor is further adapted to use the image analysis to identify if any of a predetermined set of identifying markings is present.

3. The system of claim 1 or 2, wherein the processor is adapted to determine a bottle type using a neural network.

4. The system of any one of claims 1 to 3, wherein the processor is adapted to use image analysis to identify a liquid surface.

5. The system of any one of claims 1 to 4, wherein the processor is adapted to process first image data and second image data and determine a change in liquid volume.

6. The system of claim 5, for monitoring bottle feeding of an infant, wherein the changes in liquid volume correspond to milk consumption volumes.

7. The system of claim 6, further comprising a memory for storing historic milk consumption volumes.

8. The system of claim 6 or 7, wherein the processor is adapted to determine an amount of milk to be fed to an infant based on the historic milk consumption volumes, and output the determined amount.

9. The system of claim 8, wherein the processor is adapted to output the determined amount by generating an augmented reality image of the bottle which represents the determined amount of milk.

10. The system of any one of claims 1 to 9, comprising a camera for capturing the image data, as one or more images or a video stream.

11. A method of an analyzing a bottle, comprising:
receiving image data of the bottle to be analyzed;
using image analysis to identify at least shape features of the bottle, and thereby determine a bottle type;
obtaining further shape characteristics of the bottle by accessing a database which relates different bottle types to said further shape characteristics; and
using image analysis to determine a liquid level in the bottle and thereby determine a liquid volume in the bottle based on the bottle type and said further shape characteristics.

12. The method of claim 11, comprising:
determining a bottle type using a neural network; and
using image analysis identify a liquid surface.

13. The method of claim 11 or 12 for monitoring bottle feeding of an infant, comprising processing first image data and second image data and determining a change in liquid volume which corresponds to milk consumption volumes, wherein the method further comprises determining an amount of milk to be fed to an infant based on historic milk consumption volumes, and outputting the determined amount.

14. The method of claim 13, comprising outputting the determined amount by generating an augmented reality image of the bottle which represents the determined amount of milk.

15. A computer program comprising computer program code which is adapted, when said program is run on a processor, to implement the method of any one of claims 11 to 14.
